(19)

Europäisches
Patentamt

European
Patent Office

Office européen
des brevets

(11) **EP 4 029 492 A1**

(12) **EUROPEAN PATENT APPLICATION**

(43) Date of publication:
**20.07.2022  Bulletin 2022/29**

(21) Application number: **21305029.7**

(22) Date of filing: **13.01.2021**

(51) International Patent Classification (IPC):
***A61K 8/02*** (2006.01)    ***A61K 8/19*** (2006.01)
***A61Q 19/08*** (2006.01)

(52) Cooperative Patent Classification (CPC):
**A61K 8/19; A61K 8/025; A61Q 19/08**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **ImerTech SAS
75015 Paris (FR)**

(72) Inventors:
• **Esposito, Anaëlle
  13280 Raphèle-les-Arles (FR)**
• **Remia, Elodie
  31300 Toulouse (FR)**
• **Jakob, Alexandra
  31300 Toulouse (FR)**
• **Raboteau, Cédric
  13270 Fos sur Mer (FR)**

(74) Representative: **Haseltine Lake Kempner LLP
Redcliff Quay
120 Redcliff Street
Bristol BS1 6HU (GB)**

(54) **ALKALINE EARTH METAL CARBONATES FOR COSMETIC COMPOSITIONS**

(57)    An alkaline earth metal carbonate, for use in a cosmetic composition, has a BET specific surface area of at least about 60 $m^2$/g. Particles of the alkaline earth metal carbonate are substantially spherical.

**EP 4 029 492 A1**

# EP 4 029 492 A1

**Description**

## TECHNICAL FIELD

The present disclosure concerns alkaline earth metal carbonates, cosmetic compositions comprising alkaline earth metal carbonates, uses of alkaline earth metal carbonates, and methods of reducing the appearance of skin imperfections.

## BACKGROUND

Cosmetic compositions such as makeup can be used to improve the appearance of skin, for example, by reducing the appearance of skin imperfections such as wrinkles. One way of reducing the appearance of skin imperfections, known as the soft focus effect, involves a scattered reflection of light incident on the skin. The soft focus effect of cosmetic compositions can blur unevenness, wrinkles and other skin imperfections, rendering them less visible. Cosmetic compositions typically include synthetic soft focus agents, such as poly(methyl methacrylate), nylon or synthetic silica to achieve the soft focus effect.

## SUMMARY OF THE INVENTION

According to a first aspect, there is provided an alkaline earth metal carbonate for use in a cosmetic composition, the alkaline earth metal carbonate having a BET specific surface area of at least about 60 $m^2/g$, wherein particles of the alkaline earth metal carbonate are substantially spherical.

According to a second aspect, there is provided a cosmetic composition comprising the alkaline earth metal carbonate according to the first aspect and at least one other component.

According to a third aspect, there is provided a use of an alkaline earth metal carbonate having a BET specific surface area of at least about 60 $m^2/g$ in a cosmetic composition to improve the soft focus effect of the cosmetic composition, wherein particles of the alkaline earth metal carbonate are substantially spherical.

According to a fourth aspect, there is provided a method of reducing the appearance of skin imperfections, such as wrinkles, the method comprising applying (a) the alkaline earth metal carbonate according to the first aspect, or (b) the cosmetic composition according to the second aspect, to the skin.

According to a fifth aspect, there is provided an alkaline earth metal carbonate for use in a cosmetic composition, the alkaline earth metal carbonate having a BET specific surface area of at least about 60 $m^2/g$ and an agglomeration rate of at least about 85 %, for example, at least about 90 %, or at least about 92 %, the agglomeration rate A being defined by

$$A = \frac{D_{50} @ T_{90}}{D_{50} @ T_{45}} \times 100\%.$$

According to a sixth aspect, there is provided a cosmetic composition comprising the alkaline earth metal carbonate according to the fifth aspect and at least one other component.

According to a seventh aspect, there is provided a use of an alkaline earth metal carbonate having a BET specific surface area of at least about 60 $m^2/g$ in a cosmetic composition to improve the soft focus effect of the cosmetic composition, wherein the alkaline earth metal carbonate has an agglomeration rate of at least about 85 %, for example, at least about 90 %, or at least about 92 %, the agglomeration rate A being defined by

$$A = \frac{D_{50} @ T_{90}}{D_{50} @ T_{45}} \times 100\%.$$

According to an eighth aspect, there is provided a method of reducing the appearance of skin imperfections, such as wrinkles, the method comprising applying (a) the alkaline earth metal carbonate according to the fifth aspect, or (b) the cosmetic composition according to the sixth aspect, to the skin.

The skilled person will appreciate that, except where mutually exclusive, a feature described in relation to any one of the above aspects may be applied *mutatis mutandis* to any other aspect. Furthermore, except where mutually exclusive, any feature described herein may be applied to any aspect and/or combined with any other feature described herein.

2

**FIGURES**

Embodiments will now be described by way of example only, with reference to the Figures, in which:

**Figure 1** is a Scanning Electron Microscope (SEM) image of particles of example PCC1;

**Figure 2** is an SEM image of particles of example PCC9;

**Figure 3** is an SEM image of particles of example PCC13;

**Figure 4** is an SEM image of particles of example PCC4;

**Figure 5** is an SEM image of particles of example PCC6;

**Figure 6** is an SEM image of particles of example PCC7;

**Figure 7** is a plot of BET surface area as a function of soft focus effect score for various PCCs;

**Figure 8** is a plot of $D_{50}$ @ $T_{90}$ as a function of soft focus effect score for various PCCs; and

**Figure 9** is a plot of agglomeration rate as a function of soft focus effect score for various PCCs.

**DETAILED DESCRIPTION OF THE INVENTION**

It has surprisingly been found that alkaline earth metal carbonates having a high BET specific surface area (typically of at least about 60 $m^2$/g) can improve the soft focus effect of cosmetic compositions.

*Alkaline earth metal carbonates*

The alkaline earth metal carbonate may be a carbonate of a single alkaline earth metal. Alternatively, the alkaline earth metal carbonate may comprise carbonates of two or more different alkaline earth metals. The alkaline earth metal carbonate (for example, each alkaline earth metal carbonate) may comprise carbonates of one or more of the following: beryllium, magnesium, calcium, strontium, barium, radium.

The alkaline earth metal carbonate may be calcium carbonate. The alkaline earth metal carbonate may be precipitated calcium carbonate (PCC). Precipitated calcium carbonate (PCC) may be made by any method known in the art.

TAPPI Monograph Series No 30, "Paper Coating Pigments", pages 34-35, the contents of which are incorporated herein by reference, describes three main commercial processes for preparing precipitated calcium carbonate. In all three processes, limestone is first calcined to produce quicklime, and the quicklime is then slaked in water to yield calcium hydroxide or milk of lime. In the first process, the milk of lime is directly carbonated with carbon dioxide gas. This process has the advantage that little or no byproduct is formed, and it is relatively easy to control the properties and purity of the calcium carbonate product. In the second process, the milk of lime is contacted with soda ash to produce, by double decomposition, a precipitate of calcium carbonate and a solution of sodium hydroxide. Typically, the sodium hydroxide is substantially completely separated from the calcium carbonate. In the third main commercial process, the milk of lime is first contacted with ammonium chloride to give a calcium chloride solution and ammonia gas. The calcium chloride solution is then contacted with soda ash to produce, by double decomposition, precipitated calcium carbonate and a solution of sodium chloride.

Alternatively, PCC may be made by reacting gypsum (calcium sulphate) with ammonium carbonate or ammonium bicarbonate.

Alternatively, PCC may be made by reacting calcium chloride with sodium carbonate or ammonium carbonate.

Alternatively, PCC may be obtained by carbonation of milk of lime in the presence of a crystallization controller selected from: polyacrylic acid, an aminopolycarboxylic acid (e.g. EDTA), citric acid, and salts thereof; aluminium sulfate; saccharose (e.g. sucrose); and any mixtures thereof. For instance, PCC may be prepared by one or more of the methods described in WO03/004414, the contents of which are incorporated herein by reference, particularly page 2, line 11 to page 3, line 38; page 4, line 29 to page 5, line 6; and page 5, line 36 to page 6, line 28 as well as examples 4 and 5.

In the preparation process, the concentration of calcium hydroxide in the milk of lime can have a value of from about 0.3 wt. % to about 30.0 wt. %, for example, at least about 1.0 wt. %, or at least about 2.0 wt. %, or at least about 2.5 wt. %, or at least about 4.0 wt. %, or at least about 5.0 wt. %, or at least about 6.0 wt. %, or at least about 7.0 wt.

%, or at least about 8.0 wt. %, based on the weight of the milk of lime. It is recommended that the concentration of calcium hydroxide in the milk of lime does not exceed about 25.0 wt. %, for example, about 20.0 wt. %, or about 16.0 wt. %, or about 10 wt. %, or about 9 wt. %. For instance, the concentration of calcium hydroxide in the milk of lime might be from about 0.3 wt. % to about 30.0 wt.%, for example, from about 1.0 wt. % to about 30.0 wt. %, or from about 2.0 wt. % to about 30.0 wt. %, or from about 2.5 wt. % to about 30.0 wt. %, or from about 4.0 wt. % to about 30.0 wt. %, or from about 5.0 wt. % to about 30.0 wt. %, or from about 6.0 wt. % to about 30.0 wt. %, or from about 7.0 wt. % to about 30.0 wt. %, or from about 8.0 wt. % to about 30.0 wt. %, or from about 0.3 wt. % to about 20.0 wt.%, or from about 1.0 wt. % to about 20.0 wt. %, or from about 2.0 wt. % to about 20.0 wt. %, or from about 2.5 wt. % to about 20.0 wt. %, or from about 4.0 wt. % to about 20.0 wt. %, or from about 5.0 wt. % to about 20.0 wt. %, or from about 6.0 wt. % to about 20.0 wt. %, or from about 7.0 wt. % to about 20.0 wt. %, or from about 8.0 wt. % to about 20.0 wt. %, or from about 0.3 wt. % to about 16.0 wt.%, or from about 1.0 wt. % to about 16.0 wt. %, or from about 2.0 wt. % to about 16.0 wt. %, or from about 2.5 wt. % to about 16.0 wt. %, or from about 4.0 wt. % to about 16.0 wt. %, or from about 5.0 wt. % to about 16.0 wt. %, or from about 6.0 wt. % to about 16.0 wt. %, or from about 7.0 wt. % to about 16.0 wt. %, or from about 8.0 wt. % to about 16.0 wt. %, or from about 0.3 wt. % to about 10.0 wt.%, or from about 1.0 wt. % to about 10.0 wt. %, or from about 2.0 wt. % to about 10.0 wt. %, or from about 2.5 wt. % to about 10.0 wt. %, or from about 4.0 wt. % to about 10.0 wt. %, or from about 5.0 wt. % to about 10.0 wt. %, or from about 6.0 wt. % to about 10.0 wt. %, or from about 7.0 wt. % to about 10.0 wt. %, or from about 8.0 wt. % to about 10.0 wt. %, or from about 0.3 wt. % to about 9.0 wt.%, or from about 1.0 wt. % to about 9.0 wt. %, or from about 2.0 wt. % to about 9.0 wt. %, or from about 2.5 wt. % to about 9.0 wt. %, or from about 4.0 wt. % to about 9.0 wt. %, or from about 5.0 wt. % to about 9.0 wt. %, or from about 6.0 wt. % to about 9.0 wt. %, or from about 7.0 wt. % to about 9.0 wt. %, or from about 8.0 wt. % to about 9.0 wt. %, or about 8.0 wt. %.

In said preparation process, the temperature may vary from about 0°C to about 80°C, for example, from about 10°C to about 60°C. Usually, the temperature at the beginning of carbonation is equal to or higher than about 10°C, for example, equal to or higher than about 12°C. The temperature at the beginning of carbonation is typically equal to or lower than about 30°C, for example, equal to or lower than about 25°C, or equal to or lower than about 20°C. The temperature at the beginning of carbonation might for instance be about 12°C, or about 15°C, or about 18°C. The temperature at the end of carbonation might be higher, for example, from about 10°C to about 80°C, or from about 15°C to about 65°C, or from about 35°C to about 65°C.

In the preparation process, milk of lime is carbonated by reaction of the latter with carbon dioxide gas. Carbon dioxide gas having a concentration of carbon dioxide varying from about 3.0 % to about 100 % could be used with success. However, it is typical to use carbon dioxide gas for which the concentration is from about 10 % to 60 %, for example, from about 15 % to about 40 %, or from about 20 % to about 30 %, the carbon dioxide gas being diluted with air.

Some additives might also be further added during the carbonation step, such as isoascorbic acid, to increase brightness (for example, reduce yellowness) of the resulting calcium carbonate particles. Said preparation process typically leads to a precipitated calcium carbonate slurry comprising for instance about 3.0 wt. % to about 25.0 wt. % of PCC, based on the weight of the slurry.

Precipitated calcium carbonate particles might be filtered, for example through a planar filter, and dried, for instance in an oven, by spraying into a stream of hot air (spray drying), or by the action of radiation such as infrared radiation (epiradiator), typically in an oven or by the action of radiation such as infrared radiation. The resulting particles might then be further milled, for instance in a pin mill apparatus.

Such processes for making PCC typically result in very pure calcium carbonate crystals, which may be referred to as "elementary" or "primary" particles or crystallites. In this context, the terms "elementary particle", "elementary crystallite", "primary particle" or "primary crystallite" refer to a physically and chemically autonomous entity. The elementary or primary particles or crystallites may have a variety of different shapes and sizes, depending on the specific reaction process that is used. Mixtures of different morphologies may also be used.

The main polymorphs of PCC crystals are aragonite and calcite. Aragonite crystals are needle-shaped and may be randomly aggregated. Calcite crystals may be pseudo-spherical, cubic or scalenohedral in morphology. For example, calcite crystals may have rhombohedral morphology.

BET particle surface area may be measured according to the BET method, AFNOR standard X11-621 and 622 or ISO 9277. The alkaline earth metal carbonate, for example, calcium carbonate (e.g., PCC), typically has a BET specific surface area of at least about 60 $m^2/g$. The alkaline earth metal carbonate may have a BET specific surface area of at least about 64 $m^2/g$, for example, at least about 65 $m^2/g$, or at least about 70 $m^2/g$, or at least about 75 $m^2/g$, or at least about 80 $m^2/g$. The alkaline earth metal carbonate, for example, calcium carbonate (e.g., PCC), may have a BET specific surface area of no greater than about 200 $m^2/g$, for example, no greater than about 150 $m^2/g$, or no greater than about 140 $m^2/g$, or no greater than about 120 $m^2/g$, or no greater than about 100 $m^2/g$. The alkaline earth metal carbonate, for example, calcium carbonate (e.g., PCC), may have a BET specific surface area of from about 60 $m^2/g$ to about 200 $m^2/g$, for example, from about 64 $m^2/g$ to about 200 $m^2/g$, or from about 65 $m^2/g$ to about 200 $m^2/g$, or from about 70 $m^2/g$ to about 200 $m^2/g$, or from about 75 $m^2/g$ to about 200 $m^2/g$, or from about 80 $m^2/g$ to about 200 $m^2/g$, or from about 60 $m^2/g$ to about 150 $m^2/g$, or from about 64 $m^2/g$ to about 150 $m^2/g$, or from about 65 $m^2/g$ to about 150 $m^2/g$,

or from about 70 m$^2$/g to about 150 m$^2$/g, or from about 75 m$^2$/g to about 150 m$^2$/g, or from about 80 m$^2$/g to about 150 m$^2$/g, or from about 60 m$^2$/g to about 140 m$^2$/g, or from about 64 m$^2$/g to about 140 m$^2$/g, or from about 65 m$^2$/g to about 140 m$^2$/g, or from about 70 m$^2$/g to about 140 m$^2$/g, or from about 75 m$^2$/g to about 140 m$^2$/g, or from about 80 m$^2$/g to about 140 m$^2$/g, or from about 60 m$^2$/g to about 120 m$^2$/g, or from about 64 m$^2$/g to about 120 m$^2$/g, or from about 65 m$^2$/g to about 120 m$^2$/g, or from about 70 m$^2$/g to about 120 m$^2$/g, or from about 75 m$^2$/g to about 120 m$^2$/g, or from about 80 m$^2$/g to about 120 m$^2$/g, or from about 60 m$^2$/g to about 100 m$^2$/g, or from about 64 m$^2$/g to about 100 m$^2$/g, or from about 65 m$^2$/g to about 100 m$^2$/g, or from about 70 m$^2$/g to about 100 m$^2$/g, or from about 75 m$^2$/g to about 100 m$^2$/g, or from about 80 m$^2$/g to about 100 m$^2$/g.

Particle size properties may be measured in a well-known manner by sedimentation of the particulate filler or material in a fully dispersed condition in an aqueous medium using a Sedigraph 5100 machine as supplied by Micromeritics Instruments Corporation, Norcross, Georgia, USA (web-site: www.micromeritics.com), referred to herein as a "Micromeritics Sedigraph 5100 unit". Such a machine provides measurements and a plot of the cumulative percentage by weight of particles having a size, referred to in the art as the 'equivalent spherical diameter' (e.s.d), less than given e.s.d values. The mean particle size $D_{50}$ is the value determined in this way of the particle e.s.d at which there are 50% by weight of the particles which have an equivalent spherical diameter less than that $D_{50}$ value. The $D_{98}$, $D_{90}$ and the $D_{10}$ are the values determined in this way of the particle e.s.d. at which there are 98%, 90% and 10% respectively by weight of the particles which have an equivalent spherical diameter less than that $D_{98}$, $D_{90}$ or $D_{10}$ value. Particle size properties may also be measured by wet Malvern laser scattering (standard ISO 13320-1). In this technique, the size of particles in powders, suspensions and emulsions may be measured using the diffraction of a laser beam, based on the application of Mie theory. Such a machine, for example a Malvern Mastersizer S or a Malvern Mastersizer 2000 (as supplied by Malvern instruments) provides measurements and a plot of the cumulative percentage by volume of particles having a size, referred to in the art as the "equivalent spherical diameter" (e.s.d), less than given e.s.d values. The mean particle size $D_{50}$ is the value determined in this way of the particle e.s.d. at which there are 50% by volume of the particles which have an equivalent spherical diameter less than that $D_{50}$ value. For the avoidance of doubt, the measurement of particle size using laser light scattering is not an equivalent method to the sedimentation method referred to above.

The dispersion behaviour of a particulate material (such as an agglomerated particulate material) can be characterised by the parameters $D_{50}$ @ $T_{45}$ and/or $D_{50}$ @ $T_{90}$. $D_{50}$ @ $T_{45}$ and $D_{50}$ @ $T_{90}$ can be determined by a method known as "dispersion evaluation by laser method" (DELM) using laser granulometry, for example, using the Malvern Mastersizer 2000 (as supplied by Malvern instruments). The DELM method for alkaline earth metal carbonates is based on the laser granulometry method for precipitated silica described in United States Patent No. 6,610,261 by Michelin. In the DELM method, the measurement cell of a wet Malvern laser scattering device (e.g. the Malvern Mastersizer 2000) is filled with a mixture of isopropanol and a small amount of alkaline earth metal carbonate. The obscuration is then measured and should be between 1 and 3 % for the measurement to be reliable. The mixture is then subjected to mechanical stirring at about 1500 rpm for about 4 minutes and 30 seconds. The particle size distribution (PSD) is measured 45 times during this time. The 45th PSD, or the nearest representative PSD, is recorded. A PSD is considered as representative when it does not comprise agglomerates above 500 $\mu$m which could be attributed to air bubbles. The $D_{50}$ value obtained from the recorded PSD after stirring the mixture is known as the $D_{50}$ @ $T_{45}$.

Following measurement of $D_{50}$ @ $T_{45}$, the stirred mixture is subjected to pulsed ultrasound for about 4 minutes and 30 seconds at a total intensity of about 1000 J to 1500 J. The PSD is again measured 45 times during this time. The 45th PSD, or the nearest representative PSD, is recorded. A PSD is considered as representative when it does not comprise agglomerates above 500 $\mu$m which could be attributed to air bubbles. The $D_{50}$ value obtained from the recorded PSD after application of the pulsed ultrasound is known as the $D_{50}$ @ $T_{90}$. The pulsed ultrasound may be applied using a sonicator, for example, the Vibracell 75186 sonicator (available from Thermo Fisher Scientific, Inc., USA), operated at a power of 130 W and a frequency of 20 kHz, with a probe having a 3 mm stepped tip. The intensity of the pulsed ultrasound is selected by measuring a PCC reference sample and ensuring that the $D_{50}$ @ $T_{90}$ is +/- 10 % of what has been obtained previously.

The alkaline earth metal carbonate, for example, calcium carbonate (e.g., PCC), may have a $D_{50}$ @ $T_{90}$ no less than about 6 $\mu$m, for example, no less than about 7 $\mu$m, or no less than about 8 $\mu$m, or no less than about 9 $\mu$m, or no less than about 10 $\mu$m. The alkaline earth metal carbonate, for example, calcium carbonate (e.g., PCC), may have a $D_{50}$ @ $T_{90}$ no greater than about 20 $\mu$m, for example, no greater than about 19 $\mu$m, or no greater than about 18 $\mu$m, or no greater than about 17 $\mu$m, or no greater than about 16 $\mu$m, or no greater than about 15 $\mu$m. The alkaline earth metal carbonate, for example, calcium carbonate (e.g., PCC), may have a $D_{50}$ @ $T_{90}$ from about 6 $\mu$m to about 20 $\mu$m, for example, from about 7 $\mu$m to about 20 $\mu$m, or from about 8 $\mu$m to about 20 $\mu$m, or from about 9 $\mu$m to about 20 $\mu$m, or from about 10 $\mu$m to about 20 $\mu$m, or from about 6 $\mu$m to about 19 $\mu$m, or from about 7 $\mu$m to about 19 $\mu$m, or from about 8 $\mu$m to about 19 $\mu$m, or from about 9 $\mu$m to about 19 $\mu$m, or from about 10 $\mu$m to about 19 $\mu$m, or from about 6 $\mu$m to about 18 $\mu$m, or from about 7 $\mu$m to about 18 $\mu$m, or from about 8 $\mu$m to about 18 $\mu$m, or from about 9 $\mu$m to about 18 $\mu$m, or from about 10 $\mu$m to about 18 $\mu$m, or from about 6 $\mu$m to about 17 $\mu$m, or from about 7 $\mu$m to about 17 $\mu$m, or from about 8 $\mu$m to about 17 $\mu$m, or from about 9 $\mu$m to about 17 $\mu$m, or from about 10 $\mu$m to about 17 $\mu$m,

or from about 6 μm to about 16 μm, or from about 7 μm to about 16 μm, or from about 8 μm to about 16 μm, or from about 9 μm to about 16 μm, or from about 10 μm to about 16 μm, or from about 6 μm to about 15 μm, or from about 7 μm to about 15 μm, or from about 8 μm to about 15 μm, or from about 9 μm to about 15 μm, or from about 10 μm to about 15 μm.

The alkaline earth metal carbonate, for example, calcium carbonate (e.g., PCC), may have a $D_{50}$ @ $T_{45}$ no less than about 5 μm, for example, no less than about 7 μm, or no less than about 10 μm. The alkaline earth metal carbonate, for example, calcium carbonate (e.g., PCC), may have a $D_{50}$ @ $T_{45}$ no greater than about 40 μm, for example, no greater than about 30 μm, or no greater than about 20 μm, or no greater than about 15 μm. The alkaline earth metal carbonate, for example, calcium carbonate (e.g., PCC), may have a $D_{50}$ @ $T_{45}$ from about 5 μm to about 40 μm, for example, from about 7 μm to about 40 μm, or from about 10 μm to about 40 μm, or from about 5 μm to about 30 μm, or from about 7 μm to about 30 μm, or from about 10 μm to about 30 μm, or from about 5 μm to about 20 μm, or from about 7 μm to about 20 μm, or from about 10 μm to about 20 μm, or from about 5 μm to about 15 μm, or from about 7 μm to about 15 μm, or from about 10 μm to about 15 μm.

The alkaline earth metal carbonate, for example, calcium carbonate (e.g., PCC), may have an agglomeration rate of at least about 85 %, for example, at least about 90 %, or at least about 92 %, or at least about 95 %, or at least about 99 %, the agglomeration rate A being defined by

$$A = \frac{D_{50} @ T_{90}}{D_{50} @ T_{45}} \times 100\%.$$

The agglomeration rate of the alkaline earth metal carbonate, for example, calcium carbonate (e.g., PCC), may be no greater than about 102 %, for example, no greater than about 100 %, or no greater than about 99 %, or no greater than about 95 %. The agglomeration rate of the alkaline earth metal carbonate, for example, calcium carbonate (e.g., PCC), may be from about 85 % to about 102 %, for example, from about 90 % to about 102 %, or from about 92 % to about 102 %, or from about 95 % to about 102 %, or from about 99 % to about 102 %, or from about 85 % to about 100 %, or from about 90 % to about 100 %, or from about 92 % to about 100 %, or from about 95 % to about 100 %, or from about 99 % to about 100 %, or from about 85 % to about 99 %, or from about 90 % to about 99 %, or from about 92 % to about 99 %, or from about 85 % to about 95 %, or from about 90 % to about 95 %, or from about 92 % to about 95 %.

It may be that the alkaline earth metal carbonate, for example, calcium carbonate (e.g., PCC), has: a BET specific surface area from about 60 m²/g to about 200 m²/g, for example, from about 64 m²/g to about 200 m²/g, or from about 65 m²/g to about 200 m²/g, or from about 70 m²/g to about 200 m²/g, or from about 75 m²/g to about 200 m²/g, or from about 80 m²/g to about 200 m²/g, or from about 60 m²/g to about 150 m²/g, or from about 64 m²/g to about 150 m²/g, or from about 65 m²/g to about 150 m²/g, or from about 70 m²/g to about 150 m²/g, or from about 75 m²/g to about 150 m²/g, or from about 80 m²/g to about 150 m²/g, or from about 60 m²/g to about 140 m²/g, or from about 64 m²/g to about 140 m²/g, or from about 65 m²/g to about 140 m²/g, or from about 70 m²/g to about 140 m²/g, or from about 75 m²/g to about 140 m²/g, or from about 80 m²/g to about 140 m²/g, or from about 60 m²/g to about 120 m²/g, or from about 64 m²/g to about 120 m²/g, or from about 65 m²/g to about 120 m²/g, or from about 70 m²/g to about 120 m²/g, or from about 75 m²/g to about 120 m²/g, or from about 80 m²/g to about 120 m²/g, or from about 60 m²/g to about 100 m²/g, or from about 64 m²/g to about 100 m²/g, or from about 65 m²/g to about 100 m²/g, or from about 70 m²/g to about 100 m²/g, or from about 75 m²/g to about 100 m²/g, or from about 80 m²/g to about 100 m²/g; a $D_{50}$ @ $T_{90}$ from about 6 μm to about 20 μm, for example, from about 7 μm to about 20 μm, or from about 8 μm to about 20 μm, or from about 9 μm to about 20 μm, or from about 10 μm to about 20 μm, or from about 6 μm to about 19 μm, or from about 7 μm to about 19 μm, or from about 8 μm to about 19 μm, or from about 9 μm to about 19 μm, or from about 10 μm to about 19 μm, or from about 6 μm to about 18 μm, or from about 7 μm to about 18 μm, or from about 8 μm to about 18 μm, or from about 9 μm to about 18 μm, or from about 10 μm to about 18 μm, or from about 6 μm to about 17 μm, or from about 7 μm to about 17 μm, or from about 8 μm to about 17 μm, or from about 9 μm to about 17 μm, or from about 10 μm to about 17 μm, or from about 6 μm to about 16 μm, or from about 7 μm to about 16 μm, or from about 8 μm to about 16 μm, or from about 9 μm to about 16 μm, or from about 10 μm to about 16 μm, or from about 6 μm to about 15 μm, or from about 7 μm to about 15 μm, or from about 8 μm to about 15 μm, or from about 9 μm to about 15 μm, or from about 10 μm to about 15 μm; and an agglomeration rate, as defined hereinabove, from about 85 % to about 102 %, for example, from about 90 % to about 102 %, or from about 92 % to about 102 %, or from about 95 % to about 102 %, or from about 99 % to about 102 %, or from about 85 % to about 100 %, or from about 90 % to about 100 %, or from about 92 % to about 100 %, or from about 95 % to about 100 %, or from about 99 % to about 100 %, or from about 85 % to about 99 %, or from about 90 % to about 99 %, or from about 92 % to about 99 %, or from about 85 % to about 95 %, or from about 90 % to about 95 %, or from about 92 % to about 95 %.

It may be that the alkaline earth metal carbonate, for example, calcium carbonate (e.g., PCC), has: (a) a BET specific surface area from about 60 m²/g to about 200 m²/g, for example, from about 64 m²/g to about 140 m²/g; (b) a

$D_{50}$ @ $T_{90}$ of at least about 6 $\mu$m, for example, from about 6 $\mu$m to about 20 $\mu$m; and/or (c) an agglomeration rate, as defined hereinabove, of at least about 85 %, for example, at least about 90 %, or at least about 92 %.

It may be that the alkaline earth metal carbonate, for example, calcium carbonate (e.g., PCC), has: (a) a BET specific surface area from about 60 m²/g to about 100 m²/g, for example, from about 70 m²/g to about 80 m²/g; (b) a $D_{50}$ @ $T_{90}$ of at least about 10 $\mu$m, for example, from about 10 $\mu$m to about 20 $\mu$m, or from about 13 $\mu$m to about 17 $\mu$m; and/or (c) an agglomeration rate, as defined hereinabove, of least about 95 %, for example, at least about 99 %.

The alkaline earth metal carbonate, for example, calcium carbonate (e.g., PCC), typically comprises a plurality of particles. It may be that particles of the alkaline earth metal carbonate, for example, calcium carbonate (e.g., PCC), are substantially spherical (i.e. in shape). For example, it may be that no less than about 50 wt. %, for example, no less than about 60 wt. %, or no less than about 70 wt. %, or no less than about 80 wt. %, or no less than about 90 wt. %, or no less than about 95 wt. %, or no less than about 99 wt. %, of the particles of the alkaline earth metal carbonate, for example, calcium carbonate (e.g., PCC), are substantially spherical. It may be that substantially all (i.e. about 100 wt. %) of the particles of the alkaline earth metal carbonate, for example, calcium carbonate (e.g., PCC), are substantially spherical, i.e. that the alkaline earth metal carbonate, for example, calcium carbonate (e.g., PCC), consists essentially of substantially spherical particles.

It will be appreciated that the substantially spherical particles are not necessarily entirely, or precisely, spherical. For example, the substantially spherical particles may include (e.g. be) pseudo-spherical particles.

Particle sphericity may be quantified in terms of particle circularity and/or elongation. The particles of the alkaline earth metal carbonate, for example, calcium carbonate (e.g., PCC), may have a circularity (e.g. a high sensitivity (HS) circularity) of no less than about 0.8 (e.g. no less than about 0.80), for example, no less than about 0.81, or no less than about 0.82, or no less than about 0.83, or no less than about 0.84, or no less than about 0.85, or no less than about 0.86, or no less than about 0.87, or no less than about 0.88, or no less than about 0.89, or no less than about 0.9 (e.g. no less than about 0.90), or no less than about 0.91, or no less than about 0.92, or no less than about 0.93, or no less than about 0.94, or no less than about 0.95. The particles of the alkaline earth metal carbonate, for example, calcium carbonate (e.g., PCC), may have an elongation of no greater than about 0.2 (e.g. no greater than about 0.20), for example, no greater than about 0.19, or no greater than about 0.18, or no greater than about 0.17, or no greater than about 0.16, or no greater than about 0.15, or no greater than about 0.14, or no greater than about 0.13, or no greater than about 0.12, or no greater than about 0.11, or no greater than about 0.1 (e.g. no greater than about 0.10), or no greater than about 0.09, or no greater than about 0.08, or no greater than about 0.07, or no greater than about 0.06, or no greater than about 0.05.

The (HS) circularity and the elongation may be determined, for example, using a Malvern morphogranulometer (supplied by Malvern instruments).

The (HS) circularity, C, is a measure of how close a shape is to a perfect circle and defines a relationship between the area of a particle equivalent circle and the real area of a particle:

$$C = \frac{4\pi A}{P^2}$$

wherein A is the area of the particle and P is the perimeter of the particle. C takes values from 0 (irregular particle) to 1 (perfect circle).

The elongation, E, is a measure of the asymmetry of the particle shape:

$$E = 1 - \frac{W}{L}$$

wherein $W$ is the particle width and L is the particle length. E takes values from 0 (a circle or perfect square) to 1 (a needle-like, long and fine particle).

The alkaline earth metal carbonate, for example, calcium carbonate (e.g., PCC), may be a spray-dried alkaline earth metal carbonate, for example, spray-dried calcium carbonate (e.g., spray-dried PCC). The alkaline earth metal carbonate, for example, calcium carbonate (e.g., PCC), may be spray-dried using a rotary disk or through a nozzle. Spray-drying the alkaline earth metal carbonate, for example, calcium carbonate (e.g., PCC), typically produces substantially spherical particles.

It may be that the particles of the alkaline earth metal carbonate, for example, calcium carbonate (e.g., PCC), are agglomerates (e.g. substantially spherical agglomerates), each agglomerate comprising a plurality of agglomerated primary alkaline earth metal carbonate crystallites (for example, agglomerated primary calcium carbonate (e.g., PCC) crystallites).

The alkaline earth metal carbonate, for example, calcium carbonate (e.g., PCC), may be porous. The term "porous" as used in relation to alkaline earth metal carbonates, such as calcium carbonate (and, in particular, PCC), refers to the presence of voids that allow gases and/or liquids to pass through the alkaline earth metal carbonate agglomerates or aggregates, for example, calcium carbonate (e.g., PCC). In particular, the term "porous" refers to the presence of voids in between the primary crystallites which are agglomerated to form alkaline earth metal (for example, calcium carbonate (e.g., PCC)) aggregates.

The alkaline earth metal carbonate, for example, calcium carbonate (e.g., PCC), may be surface-modified. For example, the alkaline earth metal carbonate, for example, calcium carbonate (e.g., PCC), may be coated. The coating may comprise (e.g. consist (e.g. essentially) of) a silane or salt thereof, for example, an organic silane. Additionally or alternatively, the coating may comprise (e.g. consist (e.g. essentially) of) a fatty acid or salt thereof. The fatty acid or salt thereof may contain 8 to 24 carbon atoms. For example, the coating may comprise (e.g. consist (e.g. essentially) of) caprylic acid, pelargonic acid, capric acid, undecylic acid, lauric acid, tridecylic acid, myristic acid, pentadecylic acid, palmitic acid, margaric acid, stearic acid, nonadecylic acid, arachidic acid, heneicosylic acid, behenic acid, tricosylic acid, lignoceric acid, or salts thereof, or any mixture thereof. For example, the coating may comprise (e.g. consist (e.g. essentially) of) stearic acid, palmitic acid, a stearate, a palmate, or any mixture thereof. The level of coating may be from about 1 wt. % to about 20 wt. %, based on the total weight of the coated alkaline earth metal carbonate, for example, from about 1 wt. % to about 15 wt. %, or from about 1 wt. % to about 10 wt. %, or from about 1 wt. % to about 5 wt. %, or from about 2 wt. % to about 20 wt. %, or from about 2 wt. % to about 15 wt. %, or from about 2 wt. % to about 10 wt. %, or from about 2 wt. % to about 5 wt. %, or from about 3 wt. % to about 20 wt. %, or from about 3 wt. % to about 15 wt. %, or from about 3 wt. % to about 10 wt. %, or from about 3 wt. % to about 5 wt. %, or from about 3.5 wt. % to about 20 wt. %, or from about 3.5 wt. % to about 15 wt. %, or from about 3.5 wt. % to about 10 wt. %, or from about 3.5 wt. % to about 5 wt. %, or from about 4 wt. % to about 20 wt. %, or from about 4 wt. % to about 15 wt. %, or from about 4 wt. % to about 10 wt. %, or from about 4 wt. % to about 5 wt. %, or from about 5 wt. % to about 20 wt. %, or from about 5 wt. % to about 15 wt. %, or from about 5 wt. % to about 10 wt. %.

Coating the alkaline earth metal carbonate, for example, calcium carbonate (e.g., PCC), may improve the ease of application and/or the softness when a cosmetic composition comprising the alkaline earth metal carbonate, for example, calcium carbonate (e.g., PCC), is applied to the skin. Coating the alkaline earth metal carbonate, for example, calcium carbonate (e.g., PCC), with stearic acid, palmitic acid and/or salts thereof, for example, a fatty acid mixture comprising predominantly (e.g. consisting (e.g. essentially) of) stearic acid and palmitic acid, may particularly increase the ease of application and/or the softness when a cosmetic composition comprising the alkaline earth metal carbonate, for example, calcium carbonate (e.g., PCC), is applied to the skin. The alkaline earth metal carbonate, for example, calcium carbonate (e.g., PCC), may therefore be coated with from about 1 wt. % to about 20 wt. %, for example, from about 2 wt. % to about 10 wt. %, or from about 2 wt. % to about 6 wt. %, or from about 3 wt. % to about 6 wt. %, or from about 3 wt. % to about 5 wt. %, for example, about 4 wt. %, of stearic acid, palmitic acid and/or salts thereof, for example, a fatty acid mixture comprising predominantly (e.g. consisting (e.g. essentially) of) stearic acid and palmitic acid.

The term "coating" used herein is to be understood broadly, and is not limited, for example, to uniform coatings or to coatings which cover the entire surface area of a particle. Particles in which discrete regions of the surface are modified with a coating will be understood as being coated within the terms of certain embodiments of the present invention.

The alkaline earth metal carbonate, for example, calcium carbonate (e.g., PCC), may optionally further comprise one or more crystallization controller(s). The crystallization controller may, for example, be selected from the group consisting of polyacrylic acid, salts thereof and mixtures thereof, citric acid, sodium dioctylsulfosuccinate, polyaspartic acid and ethylenediaminetetraacetic acid (EDTA). The crystallization controller may, for example, be present in the alkaline earth metal carbonate, for example, calcium carbonate (e.g., PCC), in an amount no less than about 0.1 wt. % (by weight of alkaline earth metal carbonate). For example, the crystallization controller may be present in the alkaline earth metal carbonate, for example, calcium carbonate (e.g., PCC), in an amount no less than about 0.2 wt.%, or no less than about 0.25 wt. %, or no less than about 0.5 wt.%. For example, the crystallization controller may be present in the alkaline earth metal carbonate, for example, calcium carbonate (e.g., PCC), in an amount no greater than about 4 wt. %, or no greater than about 3 wt. %, or no greater than about 2.5 wt. %, or no greater than about 2 wt. %, or no greater than about 1 wt. %. Accordingly, the crystallization controller may be present in the alkaline earth metal carbonate, for example, calcium carbonate (e.g., PCC), in an amount from about 0.2 wt. % to about 4 wt. %, for example, from about 0.25 wt. % to about 4 wt. %, or from about 0.5 wt. % to about 4 wt. %, or from about 0.2 wt. % to about 3 wt. %, or from about 0.25 wt. % to about 3 wt. %, or from about 0.5 wt. % to about 3 wt. %, or from about 0.2 wt. % to about 2.5 wt. %, or from about 0.25 wt. % to about 2.5 wt. %, or from about 0.5 wt. % to about 2.5 wt. %, or from about 0.2 wt. % to about 2 wt. %, or from about 0.25 wt. % to about 2 wt. %, or from about 0.5 wt. % to about 2 wt. %.

When polyacrylic acid, salts thereof and mixtures thereof are present in the alkaline earth metal carbonate, for example, calcium carbonate (e.g., PCC), particles, generally the molecular weight of the polyacrylic acid or its salt, in particular sodium salt, is from about 500 and up to about 15,000 g/mol. The molecular weight may, for example, be equal to or larger than about 500 g/mol or equal to or larger than about 700 g/mol or equal to or larger than about 1000 g/mol. Generally, the molecular weight is equal to or less than about 15,000 g/mol, or equal to or less than about 10,000 g/mol or equal to or less than about 5000 g/mol. For example, the molecular weights may be from about 1000 to about 3500 g/mol. If the polyacrylic acid is present as salt, such as the sodium salt, the degree of acid neutralization by its cation, in particular sodium, can be from 0 to 100 %. For instance, around 70 % of the acid groups may be neutralized. The crystallization controlled may thus have a pH ranging from about 5 to about 6. For instance, around 100 % of the acid groups may be neutralized and the crystallization controller may have a pH ranging from about 6.5 to about 10.

*Cosmetic composition*

The cosmetic composition typically comprises the alkaline earth metal carbonate, for example, calcium carbonate (e.g., PCC), and at least one other component.

The cosmetic composition may comprise no less than about 0.5 wt. %, for example, no less than about 1 wt. %, or no less than about 2 wt. %, or no less than about 5 wt. %, or no less than about 10 wt. %, or no less than about 20 wt. %, of the alkaline earth metal carbonate, for example, calcium carbonate (e.g., PCC). The cosmetic composition may comprise no greater than about 90 wt. %, for example, no greater than about 80 wt. %, or no greater than about 70 wt. %, or no greater than about 60 wt. %, or no greater than about 50 wt. %, or no greater than about 40 wt. %, or no greater than about 30 wt. %, or no greater than about 20 wt. %, or no greater than about 10 wt. %, or no greater than about 5 wt. %, of the alkaline earth metal carbonate, for example, calcium carbonate (e.g., PCC). The cosmetic composition may comprise from about 0.5 wt. % to about 90 wt. %, for example, from about 1 wt. % to about 90 wt. %, or from about 2 wt. % to about 90 wt. %, or from about 5 wt. % to about 90 wt. %, or from about 10 wt. % to about 90 wt. %, or from about 20 wt. % to about 90 wt. %, or from about 0.5 wt. % to about 80 wt. %, or from about 1 wt. % to about 80 wt. %, or from about 2 wt. % to about 80 wt. %, or from about 5 wt. % to about 80 wt. %, or from about 10 wt. % to about 80 wt. %, or from about 20 wt. % to about 80 wt. %, or from about 0.5 wt. % to about 70 wt. %, or from about 1 wt. % to about 70 wt. %, or from about 2 wt. % to about 70 wt. %, or from about 5 wt. % to about 70 wt. %, or from about 10 wt. % to about 70 wt. %, or from about 20 wt. % to about 70 wt. %, or from about 0.5 wt. % to about 60 wt. %, or from about 1 wt. % to about 60 wt. %, or from about 2 wt. % to about 60 wt. %, or from about 5 wt. % to about 60 wt. %, or from about 10 wt. % to about 60 wt. %, or from about 20 wt. % to about 60 wt. %, or from about 0.5 wt. % to about 50 wt. %, or from about 1 wt. % to about 50 wt. %, or from about 2 wt. % to about 50 wt. %, or from about 5 wt. % to about 50 wt. %, or from about 10 wt. % to about 50 wt. %, or from about 20 wt. % to about 50 wt. %, or from about 0.5 wt. % to about 40 wt. %, or from about 1 wt. % to about 40 wt. %, or from about 2 wt. % to about 40 wt. %, or from about 5 wt. % to about 40 wt. %, or from about 10 wt. % to about 40 wt. %, or from about 20 wt. % to about 40 wt. %, or from about 0.5 wt. % to about 30 wt. %, or from about 1 wt. % to about 30 wt. %, or from about 2 wt. % to about 30 wt. %, or from about 5 wt. % to about 30 wt. %, or from about 10 wt. % to about 30 wt. %, or from about 20 wt. % to about 30 wt. %, or from about 0.5 wt. % to about 20 wt. %, or from about 1 wt. % to about 20 wt. %, or from about 2 wt. % to about 20 wt. %, or from about 5 wt. % to about 20 wt. %, or from about 10 wt. % to about 20 wt. %, or from about 0.5 wt. % to about 10 wt. %, or from about 1 wt. % to about 10 wt. %, or from about 2 wt. % to about 10 wt. %, or from about 5 wt. % to about 10 wt. %, or from about 0.5 wt. % to about 5 wt. %, or from about 1 wt. % to about 5 wt. %, or from about 2 wt. % to about 5 wt. %, of the alkaline earth metal carbonate, for example, calcium carbonate (e.g., PCC).

The at least one other component may be selected from: water or an aqueous solution; a polymer; an oil, such as an oil derived from a plant source, an animal source or a mineral (e.g. petrochemical) source; a wax, such as a wax derived from a plant source, an animal source or a mineral (e.g. petrochemical) source; a fatty acid or salt thereof; an amino acid or a polypeptide such as a protein; a sugar or carbohydrate; a mineral other than the alkaline earth metal carbonate, for example, an iron oxide, zinc oxide, talc, mica, wollastonite, diatomaceous earth or a clay mineral (e.g. kaolinite, montmorillonite or illite); a pigment; a perfume. The cosmetic composition may contain two or more (i.e. different components) other than the alkaline earth metal carbonate.

The cosmetic composition may be an anhydrous cosmetic composition, i.e. a cosmetic composition which is substantially (e.g. entirely) water-free. For example, the cosmetic composition may comprise no greater than about 1 wt. %, for example, no greater than about 0.1 wt. %, or no greater than about 0.01 wt. %, water. Alternatively, the cosmetic composition may be a non-anhydrous (i.e. hydrous or water-containing) cosmetic composition. For example, the cosmetic composition may comprise no less than about 1 wt. %, for example, greater than about 1 wt. %, or greater than about 5 wt. %, water.

The cosmetic composition may be a water-based cosmetic composition. Alternatively, the cosmetic composition may be an oil-based cosmetic composition. Further alternatively, the cosmetic composition may contain both water and an oil, for example, in the form of an emulsion.

The cosmetic composition may be a dry cosmetic composition. For example, the cosmetic composition may be a powder, such as a loose powder or a compressed powder.

The cosmetic composition may be a wet cosmetic composition. For example, the cosmetic composition may be a cream, gel, gel-cream, emulsion, lotion, fluid, milk or serum.

The cosmetic composition may be a solid cosmetic composition. For example, the cosmetic composition may be a solid stick.

The cosmetic composition may be a liquid cosmetic composition. For example, the cosmetic composition may be a cream, lotion, fluid, milk or serum.

The cosmetic composition may be semi-solid cosmetic composition. For example, the cosmetic composition may be a gel.

The cosmetic composition may comprise a mixture of one or more liquid components and one or more solid components. For example, the cosmetic composition may be a paste or slurry.

The cosmetic composition is typically for topical application to the skin. For example, the cosmetic composition may be for topical application to the skin of the head and neck, for example, facial skin (which may include the lips). Additionally or alternatively, the cosmetic composition may be for topical application to the skin of the body (for example, of the limbs, such as the arms or legs, and/or the décolletage).

The cosmetic composition may be, or may be suitable for use in, a form of make-up. For example, the cosmetic composition may be or may be suitable for use in: a primer; a concealer; a foundation; a rouge or a blusher; a bronzer; a highlighter; an eyeshadow; an eyebrow pencil, cream, wax, gel or powder; an eyeliner pencil, gel or liquid; a mascara; a lipstick, lip gloss, lip balm or lip liner; or a face powder, setting powder or setting spray.

Alternatively, the cosmetic composition may be, or may be suitable for use in, a form of skincare. For example, the cosmetic composition may be or may be suitable for use in: a moisturiser; a serum; a toner; a skin oil (for example, a facial oil); or a sunscreen.

*Soft focus effect*

It has been found that including the alkaline earth metal carbonate in the cosmetic composition can improve the soft focus effect of the cosmetic composition, i.e. the soft focus effect achieved when the cosmetic composition is applied to the skin.

The soft focus effect, when the cosmetic composition is applied to the skin, may reduce the appearance of (i.e. render less visible or noticeable) skin imperfections. For example, the soft focus effect may reduce the appearance of (i.e. render less visible or noticeable) wrinkles, otherwise known as rhytids, i.e. folds, ridges or creases in the skin. Additionally or alternatively, the soft focus effect may reduce the appearance of (i.e. render less visible or noticeable) blemishes such as liver spots (also known as age spots), dark circles, freckles, pimples, blackheads, whiteheads, enlarged pores, melasma, scars, hyperpigmentation and/or bruises.

The soft focus effect of the alkaline earth metal carbonate may be evaluated by trained panellists. For example, the alkaline earth metal carbonate may be incorporated into an oil phase matrix, having a refractive index which substantially matches that of the alkaline earth metal carbonate (e.g. 1.5 for calcium carbonate). Dicaprylyl carbonate is a suitable oil for testing calcium carbonate. A mixture of 50%-50% by weight alkaline earth metal carbonate to oil may be used. A measured amount of the mixture may be applied and spread over a defined area of the panellists' skin, for example in the hollow of the wrist where fine lines are visible. The panellists can rate the soft focus effect, for example on a scale of 0 (corresponding to the soft focus effect achieved using oil alone) to 5 (corresponding to the soft focus effect achieved using a reference synthetic soft focus agent such as CL-SILICA LA 300 (available from Chemland Co., Ltd.).

It will be understood that the invention is not limited to the embodiments described above and various modifications and improvements can be made without departing from the concepts described herein. Except where mutually exclusive, any of the features may be employed separately or in combination with any other features and the disclosure extends to and includes all combinations and sub-combinations of one or more features described herein.

For the avoidance of doubt, the present application is directed to the subject-matter described in the following numbered paragraphs:

1. Alkaline earth metal carbonate for use in a cosmetic composition, the alkaline earth metal carbonate having a BET specific surface area of at least about 60 $m^2/g$, wherein particles of the alkaline earth metal carbonate are substantially spherical.

2. The alkaline earth metal carbonate according to paragraph 1, wherein the alkaline earth metal carbonate is calcium carbonate, for example, precipitated calcium carbonate.

3. The alkaline earth metal carbonate according to paragraph 1 or paragraph 2, wherein the alkaline earth metal carbonate:

(a) has a BET specific surface area from about 60 m$^2$/g to about 200 m$^2$/g, for example, from about 64 m$^2$/g to about 140 m$^2$/g;

(b) has a $D_{50}$ @ $T_{90}$ of at least about 6 $\mu$m, for example, from about 6 $\mu$m to about 20 $\mu$m; and/or

(c) has an agglomeration rate of at least about 85 %, for example, at least about 90 %, or at least about 92 %, the agglomeration rate A being defined by

$$A = \frac{D_{50} @ T_{90}}{D_{50} @ T_{45}} \times 100\%.$$

4. The alkaline earth metal carbonate according to any preceding paragraph, wherein the particles of the alkaline earth metal carbonate have an HS circularity of at least about 0.8 and/or an elongation of no greater than about 0.2.

5. The alkaline earth metal carbonate according to any preceding paragraph, wherein the particles of the alkaline earth metal carbonate are agglomerates each comprising a plurality of agglomerated primary alkaline earth metal carbonate crystallites.

6. The alkaline earth metal carbonate according to any preceding paragraph, wherein the alkaline earth metal carbonate is coated, for example, with a coating comprising a fatty acid or salt thereof, such as stearic acid, palmitic acid, a stearate or a palmate, or any mixture thereof.

7. The alkaline earth metal carbonate according to paragraph 6, wherein the alkaline earth metal carbonate comprises from about 1 wt. % to about 20 wt. %, for example, from about 2 wt. % to about 15 wt. %, or from about 3.5 wt. % to about 15 wt. %, of the fatty acid or salt thereof, such as the stearic acid, palmitic acid, the stearate or the palmate, or any mixture thereof.

8. A cosmetic composition comprising the alkaline earth metal carbonate according to any preceding paragraph and at least one other component.

9. Use of an alkaline earth metal carbonate having a BET specific surface area of at least about 60 m$^2$/g in a cosmetic composition to improve the soft focus effect of the cosmetic composition, wherein particles of the alkaline earth metal carbonate are substantially spherical.

10. The use according to paragraph 9, wherein the alkaline earth metal carbonate:

(a) is calcium carbonate, for example, precipitated calcium carbonate;

(b) has a BET specific surface area from about 60 m$^2$/g to about 200 m$^2$/g, for example, from about 64 m$^2$/g to about 140 m$^2$/g;

(c) has a $D_{50}$ @ $T_{90}$ of at least about 6 $\mu$m, for example, from about 6 $\mu$m to about 20 $\mu$m; and/or

(d) has an agglomeration rate of at least about 85 %, for example, at least about 90 %, or at least about 92 %, the agglomeration rate A being defined by

$$A = \frac{D_{50} @ T_{90}}{D_{50} @ T_{45}} \times 100\%.$$

11. The use according to paragraph 9 or paragraph 10, wherein the particles of the alkaline earth metal carbonate have an HS circularity of at least about 0.8 and/or an elongation of no greater than about 0.2.

12. The use according to any of paragraphs 9 to 11, wherein the particles of the alkaline earth metal carbonate are agglomerates each comprising a plurality of agglomerated primary alkaline earth metal carbonate crystallites.

13. The use according to any of paragraphs 9 to 12, wherein the alkaline earth metal carbonate is coated, for example, with a coating comprising a fatty acid or salt thereof, such as stearic acid, palmitic acid, a stearate, or a palmate, or any mixture thereof.

14. The use according to paragraph 13, wherein the alkaline earth metal carbonate comprises from about 1 wt. % to about 10 wt. %, for example, from about 2 wt. % to about 5 wt. %, or from about 3.5 wt. % to about 4.5 wt. %, of the fatty acid or salt thereof, such as the stearic acid, palmitic acid, stearate, or palmate, or any mixture thereof.

15. A method for reducing the appearance of skin imperfections, such as wrinkles, the method comprising applying (a) the alkaline earth metal carbonate according to any of paragraphs 1 to 7, or (b) the cosmetic composition according to paragraph 8, to the skin.

**EXAMPLES**

*Example 1*

Twenty different example precipitated calcium carbonates (PCC1 to PCC20), as set out in Table 1, were obtained or prepared.
PCC1, PCC2 and PCC9 to PCC20 were prepared by the following method. A milk of lime consisting of calcium hydroxide ($Ca(OH)_2$) at a concentration of 80 g/L was placed in a 350 L carbonator. Citric acid was added at a concentration of 2 wt. % vs. PCC. Carbonation reaction was performed at 15 °C. The $CO_2$ flow rate was 90 $Nm^3$/h. When the reaction was completed and the pH reached 7, an overcarbonation was performed at 26 $Nm^3$/h to ensure that all of the $Ca(OH)_2$ had been carbonated. The resultant product was optionally functionalized with a stearin (a mixture of fatty acids, mostly palmitic and stearic acids) as set out in Table 1. If functionalised with the stearin, the fatty acid was added in emulsion with an alkali such as $NH_4OH$ or NaOH. The product was sieved to remove the bigger parts. Finally, the product was spray-dried using either a rotary disk method or through a nozzle, as set out in Table 1. For the spray-drying, the inlet temperature was 300 °C, while the outlet temperature was 105 °C.
PCC3 to PCC8 were different commercially available precipitated calcium carbonates also prepared by spray-drying. PCC2, PCC4, PCC6 and PCC8 were coated with maltodextrin.

Table 1.

| PCC | Coating | Drying method | Particle shape |
|---|---|---|---|
| PCC1 | None | Spray dried | Spherical |
| PCC2 | maltodextrin | Spray dried | Spherical |
| PCC3 | None | Spray dried | Ellipsoid |
| PCC4 | 2 wt. % maltodextrin | Spray dried | Ellipsoid |
| PCC5 | None | Spray dried | Ellipsoid |
| PCC6 | 2 wt. % maltodextrin | Spray dried | Ellipsoid |
| PCC7 | None | Spray dried | Ellipsoid |
| PCC8 | 2 wt. % maltodextrin | Spray dried | Ellipsoid |
| PCC9 | None | Spray dried | Spherical |
| PCC10 | 2 wt. % stearin | Spray dried | Spherical |
| PCC11 | None | Spray dried (nozzle) | Spherical |
| PCC 12 | 2 wt. % stearin | Spray dried (nozzle) | Spherical |
| PCC13 | 4 wt. % stearin | Spray dried (nozzle) | Spherical |
| PCC 14 | 4 wt. % stearin | Spray dried (nozzle) | Spherical |
| PCC15 | None | Spray dried (nozzle) | Spherical |
| PCC 16 | None | Spray dried (nozzle) | Spherical |
| PCC17 | None | Spray dried (nozzle) | Spherical |
| PCC18 | None | Spray dried (nozzle) | Spherical |

(continued)

| PCC | Coating | Drying method | Particle shape |
|---|---|---|---|
| PCC19 | 4 wt. % stearin | Spray dried (disk) | Spherical |
| PCC20 | 4 wt. % stearin | Spray dried (nozzle) | Spherical |

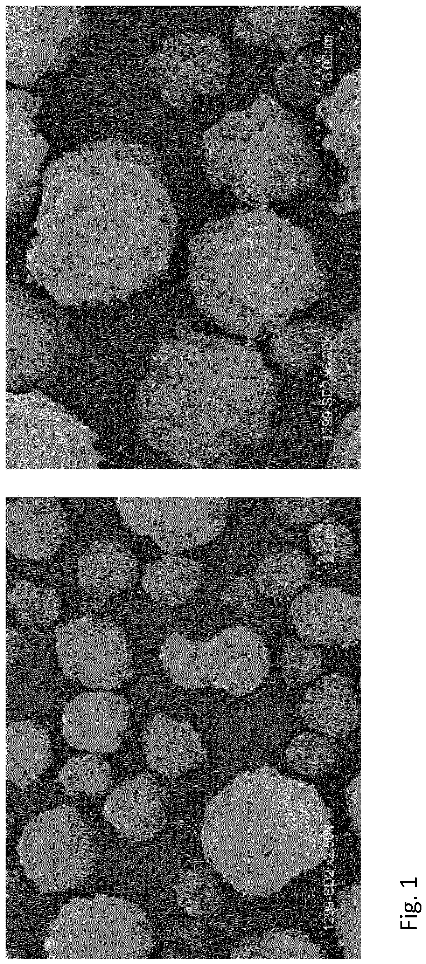

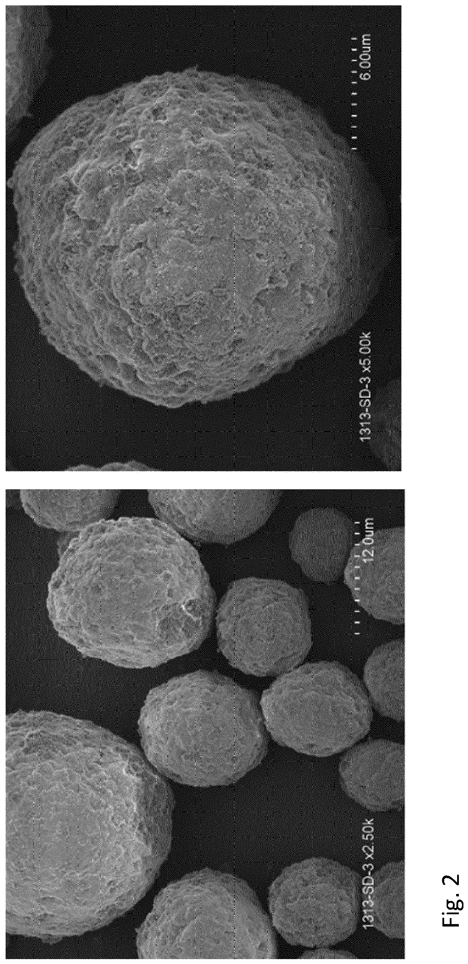

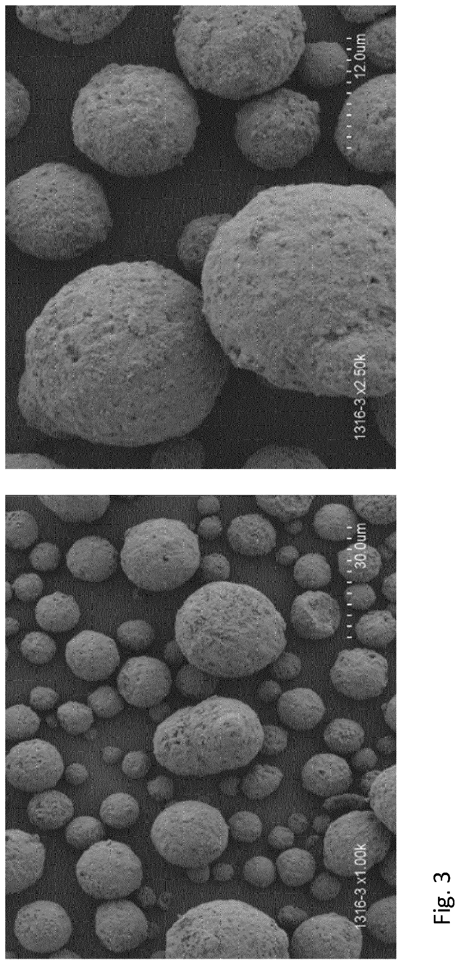

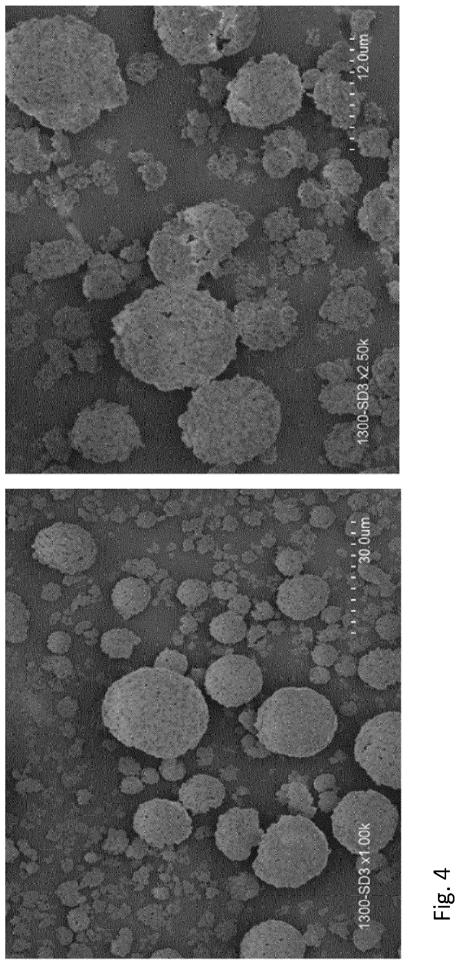

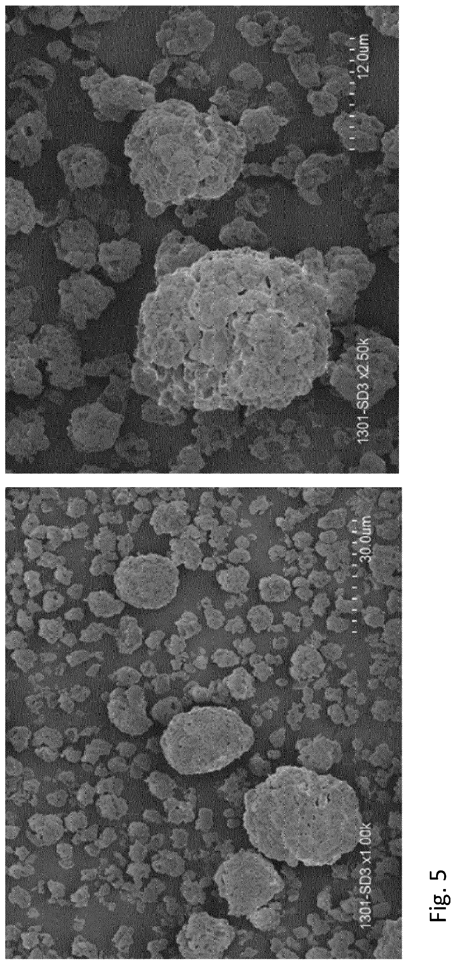

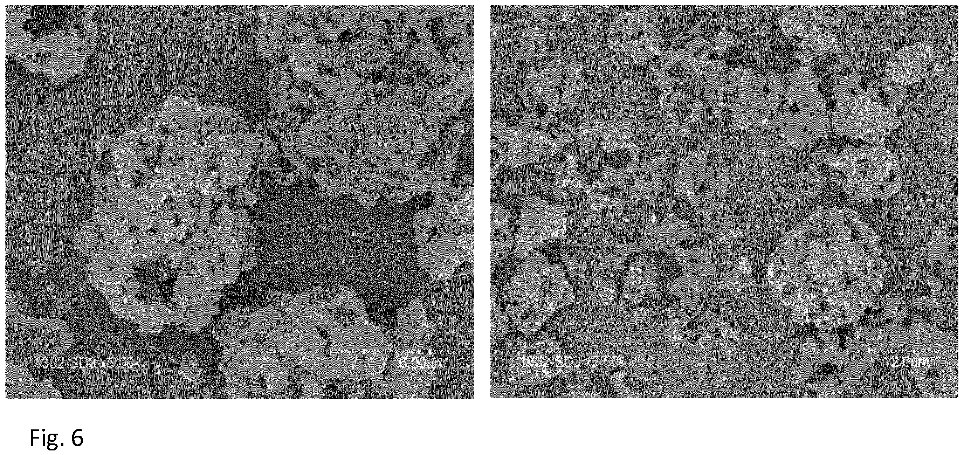

Samples of PCC1, PCC4, PCC6, PCC7, PCC9 and PCC13 were observed in a Scanning Electron Microscope (Hitachi S4800 Field Emission Scanning Electron Microscope with a secondary electron and backscattered electron detector, an accelerating voltage of 0.1 kV to 30 kV, maximum magnification of 800 000x, resolution of 1 nm at 15 kV, and a transmission option for the observation of fine samples at 30 kV) following sputtering with platinum (Pt). Figure 1 shows SEM images obtained for PCC1. Figure 2 shows SEM images obtained for PCC9. Figure 3 shows SEM images obtained for PCC13. Figure 4 shows SEM images obtained for PCC4. Figure 5 shows SEM images obtained for PCC6. Figure 6 shows SEM images obtained for PCC7.

For each of PCC1 to PCC20, BET surface area, $D_{50}$ @ $T_{90}$ and agglomeration rate were measured.

The BET surface area was measured using the BET method, AFNOR standard X11-621 and 622 or ISO 9277.

$D_{50}$ @ $T_{45}$, $D_{50}$ @ $T_{90}$ and the agglomeration rate were determined by the "dispersion evaluation by laser method" (DELM) using laser granulometry using the Malvern Mastersizer 2000 (as supplied by Malvern instruments). The measurement cell of the Malvern Mastersizer 2000 was filled, in turn, with a mixture of isopropanol and a small amount of each PCC. The obscuration was then measured and the mixture adjusted so that the obscuration was between 1 and 3 % (for the measurement to be reliable). The mixture was then subjected to mechanical stirring at about 1500 rpm for about 4 minutes and 30 seconds. The particle size distribution (PSD) was measured 45 times during this time. The 45th PSD, or the nearest representative PSD, was recorded. A PSD was considered as representative when it did not comprise agglomerates above 500 $\mu$m which could be attributed to air bubbles. The $D_{50}$ value obtained from the recorded PSD after stirring the mixture was recorded as the $D_{50}$ @ $T_{45}$.

Following measurement of $D_{50}$ @ $T_{45}$, the stirred mixture was subjected to pulsed ultrasound for about 4 minutes and 30 seconds at a total intensity of about 1000 J to 1500 J. The pulsed ultrasound was applied using the Vibracell 75186 sonicator (available from Thermo Fisher Scientific, Inc., USA), operated at a power of 130 W and a frequency of 20 kHz, with a probe having a 3 mm stepped tip. The PSD was again measured 45 times during this time. The 45th PSD, or the nearest representative PSD, was recorded. A PSD was considered as representative when it did not comprise agglomerates above 500 $\mu$m which could be attributed to air bubbles. The $D_{50}$ value obtained from the recorded PSD after application of the pulsed ultrasound was recorded as the $D_{50}$ @ $T_{90}$. The intensity of the pulsed ultrasound was selected by measuring a PCC reference sample and ensuring that the $D_{50}$ @ $T_{90}$ was +/- 10 % of what had been obtained previously.

The agglomeration rate, A, was calculated according to:

$$A = \frac{D_{50} \ @ \ T_{90}}{D_{50} \ @ \ T_{45}} \times 100\%.$$

The results are shown in Table 2.

Table 2.

| PCC | BET surface area (m²/g) | $D_{50}$ @ $T_{90}$ ($\mu$m) | Agglomeration rate (%) |
|---|---|---|---|
| PCC1 | 106 | 9.8 | 97.8 |
| PCC2 | 98 | 8.6 | 97.5 |
| PCC3 | 20 | 1.8 | 24.7 |
| PCC4 | 17 | 6.2 | 56.0 |
| PCC5 | 57 | 4.7 | 55.3 |
| PCC6 | 51 | 5.2 | 57.7 |
| PCC7 | 10 | 4.5 | 84.4 |
| PCC8 | 7 | 6.0 | 69.9 |
| PCC9 | 134 | 18.3 | 99.8 |

(continued)

| PCC | BET surface area (m²/g) | $D_{50}$ @ $T_{90}$ (µm) | Agglomeration rate (%) |
|---|---|---|---|
| PCC10 | 64 | 18.0 | 97.6 |
| PCC11 | 80 | 15.7 | 100.4 |
| PCC 12 | 78 | 16.0 | 99.3 |
| PCC13 | 76 | 15.2 | 99.7 |
| PCC14 | 78 | 16.1 | 99.3 |
| PCC15 | 76 | 9.2 | 98.5 |
| PCC16 | 77 | 9.7 | 94.1 |
| PCC17 | 80 | 13.2 | 97.9 |
| PCC18 | 80 | 8.6 | 93.7 |
| PCC19 | 69 | 8.0 | 99.4 |
| PCC20 | 68 | 15.2 | 98.6 |

The soft focus effect and ease of application of each of PCC1 to PCC20 was evaluated by a panel of 5 trained panellists by the following method. A 50%-50% mixture of each PCC with dicaprylyl carbonate oil was made. A measured amount of the mixture was applied and spread over a defined area of the panellists' skin in the hollow of the wrist where fine lines were visible. The panellists rated the soft focus effect (i.e. the ability to reduce the appearance of the fine lines) on a scale of 0 (corresponding to the soft focus effect achieved using the oil alone) to 5 (corresponding to the soft focus effect achieved using a reference synthetic soft focus agent, CL-SILICA LA 300 (available from Chemland Co., Ltd.)). The panellists also rated the ease of application on a scale of 0 (corresponding to least easy to apply) to 5 (corresponding to easiest to apply). The results are presented in Table 3.

Table 3.

| PCC | Soft focus effect (scale 0 to 5) | Ease of application (scale of 0 to 5) | Inventive? |
|---|---|---|---|
| PCC1 | 5 | 2 | YES |
| PCC2 | 5 | 3 | YES |
| PCC3 | 1 | 1 | NO |
| PCC4 | 1 | 1 | NO |
| PCC5 | 3 | 3 | NO |
| PCC6 | 3 | 3 | NO |
| PCC7 | 1 | 1 | NO |
| PCC8 | 1 | 1 | NO |
| PCC9 | 5 | 2 | YES |
| PCC10 | 5 | 5 | YES |
| PCC11 | 5 | 3 | YES |
| PCC12 | 5 | 3 | YES |
| PCC13 | 5 | 5 | YES |
| PCC14 | 5 | 5 | YES |
| PCC15 | 5 | 0 | YES |
| PCC16 | 5 | 1 | YES |
| PCC17 | 5 | 0 | YES |
| PCC18 | 5 | 2 | YES |

(continued)

| PCC | Soft focus effect (scale 0 to 5) | Ease of application (scale of 0 to 5) | Inventive? |
|---|---|---|---|
| PCC19 | 5 | 6 | YES |
| PCC20 | 5 | 5 | YES |

Based on the results shown in Table 3, PCC1, PCC2 and PCC9 to PCC20, all of which achieved a score of 5 for the soft focus effect, are considered to be inventive examples. PCC3 to PCC8, all of which achieved a score of 3 or lower for the soft focus effect, are considered to be reference examples.

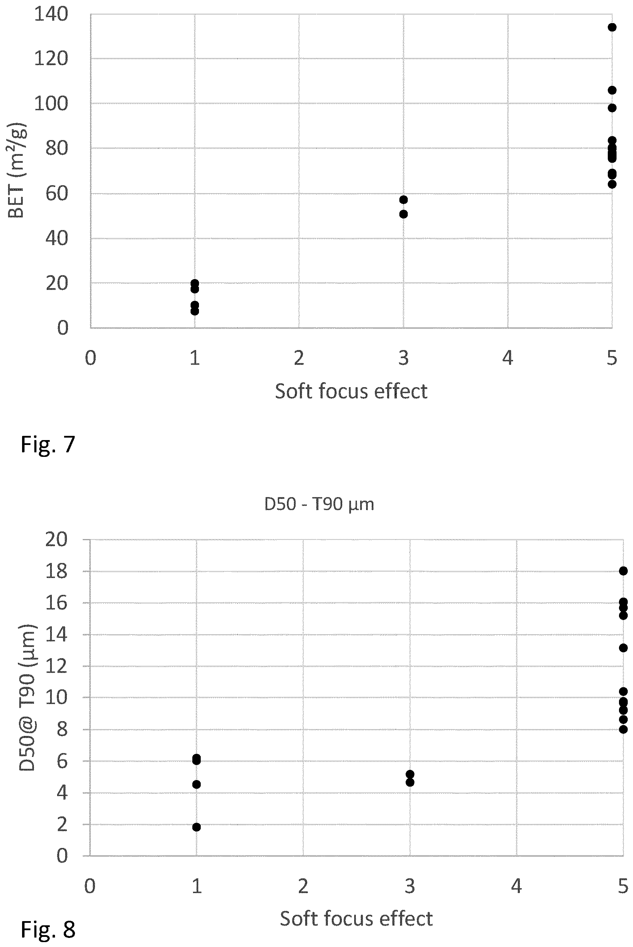

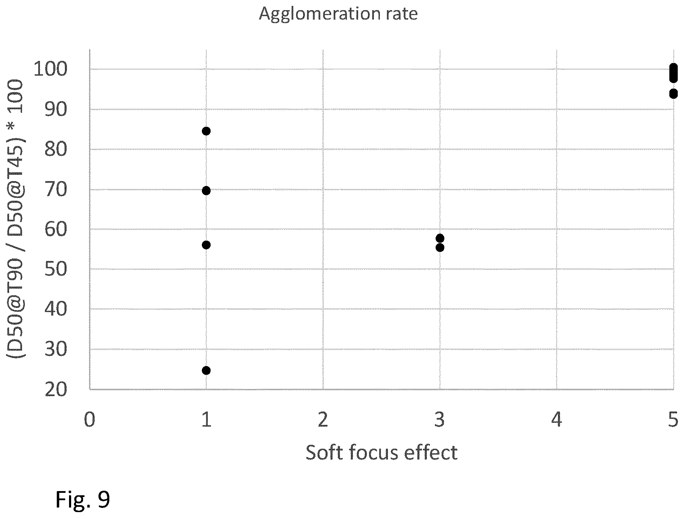

Figures 7, 8 and 9 show plots of the BET surface area, $D_{50}$ @ $T_{90}$ and agglomeration rate as a function of the soft focus effect. From these plots, it can be seen that an improved soft focus effect is correlated with higher (e.g. no less than about 60 m²/g) BET surface areas, higher (e.g. no less than about 6 $\mu$m, or no less than about 8 $\mu$m) values of $D_{50}$ @ $T_{90}$, and higher (e.g. no less than about 85 %, for example, no less than about 90 %) agglomeration rates.

By comparing the results of Tables 1 and 3, it can also be seen that an improved ease of application is correlated with use of a stearin coating, and that the best ease of application is achieved when a 4 wt. % stearin coating is used.

## Claims

1. Alkaline earth metal carbonate for use in a cosmetic composition, the alkaline earth metal carbonate having a BET specific surface area of at least about 60 m²/g, wherein particles of the alkaline earth metal carbonate are substantially spherical.

2. The alkaline earth metal carbonate according to claim 1, wherein the alkaline earth metal carbonate is calcium carbonate, for example, precipitated calcium carbonate.

3. The alkaline earth metal carbonate according to claim 1 or claim 2, wherein the alkaline earth metal carbonate:

   (a) has a BET specific surface area from about 60 m²/g to about 200 m²/g, for example, from about 64 m²/g to about 140 m²/g;
   (b) has a $D_{50}$ @ $T_{90}$ of at least about 6 $\mu$m, for example, from about 6 $\mu$m to about 20 $\mu$m; and/or
   (c) has an agglomeration rate of at least about 85 %, for example, at least about 90 %, or at least about 92 %, the agglomeration rate A being defined by

$$A = \frac{D_{50} @ T_{90}}{D_{50} @ T_{45}} \times 100\%.$$

4. The alkaline earth metal carbonate according to any preceding claim, wherein the particles of the alkaline earth metal carbonate have an HS circularity of at least about 0.8 and/or an elongation of no greater than about 0.2.

5. The alkaline earth metal carbonate according to any preceding claim, wherein the particles of the alkaline earth metal carbonate are agglomerates each comprising a plurality of agglomerated primary alkaline earth metal carbonate crystallites.

6. The alkaline earth metal carbonate according to any preceding claim, wherein the alkaline earth metal carbonate is coated, for example, with a coating comprising a fatty acid or salt thereof, such as stearic acid, palmitic acid, a stearate or a palmate, or any mixture thereof.

7. The alkaline earth metal carbonate according to claim 6, wherein the alkaline earth metal carbonate comprises from about 1 wt. % to about 20 wt. %, for example, from about 2 wt. % to about 15 wt. %, or from about 3.5 wt. % to about 15 wt. %, of the fatty acid or salt thereof, such as the stearic acid, palmitic acid, the stearate or the palmate, or any mixture thereof.

8. A cosmetic composition comprising the alkaline earth metal carbonate according to any preceding claim and at least one other component.

9. Use of an alkaline earth metal carbonate having a BET specific surface area of at least about 60 m$^2$/g in a cosmetic composition to improve the soft focus effect of the cosmetic composition, wherein particles of the alkaline earth metal carbonate are substantially spherical.

10. The use according to claim 9, wherein the alkaline earth metal carbonate:

    (a) is calcium carbonate, for example, precipitated calcium carbonate;
    (b) has a BET specific surface area from about 60 m$^2$/g to about 200 m$^2$/g, for example, from about 64 m$^2$/g to about 140 m$^2$/g;
    (c) has a $D_{50}$ @ $T_{90}$ of at least about 6 $\mu$m, for example, from about 6 $\mu$m to about 20 $\mu$m; and/or
    (d) has an agglomeration rate of at least about 85 %, for example, at least about 90 %, or at least about 92 %, the agglomeration rate A being defined by

$$A = \frac{D_{50} @ T_{90}}{D_{50} @ T_{45}} \times 100\%.$$

11. The use according to claim 9 or claim 10, wherein the particles of the alkaline earth metal carbonate have an HS circularity of at least about 0.8 and/or an elongation of no greater than about 0.2.

12. The use according to any of claims 9 to 11, wherein the particles of the alkaline earth metal carbonate are agglomerates each comprising a plurality of agglomerated primary alkaline earth metal carbonate crystallites.

13. The use according to any of claims 9 to 12, wherein the alkaline earth metal carbonate is coated, for example, with a coating comprising a fatty acid or salt thereof, such as stearic acid, palmitic acid, a stearate, or a palmate, or any mixture thereof.

14. The use according to claim 13, wherein the alkaline earth metal carbonate comprises from about 1 wt. % to about 10 wt. %, for example, from about 2 wt. % to about 5 wt. %, or from about 3.5 wt. % to about 4.5 wt. %, of the fatty acid or salt thereof, such as the stearic acid, palmitic acid, stearate, or palmate, or any mixture thereof.

15. A method for reducing the appearance of skin imperfections, such as wrinkles, the method comprising applying (a) the alkaline earth metal carbonate according to any of claims 1 to 7, or (b) the cosmetic composition according to claim 8, to the skin.

Fig. 1

Fig. 2

Fig. 3

Fig. 4

Fig. 5

Fig. 6

Fig. 7

D50 - T90 µm

Fig. 8

Agglomeration rate

Fig. 9

| | Europäisches Patentamt | **EUROPEAN SEARCH REPORT** | Application Number |
|---|---|---|---|
| | European Patent Office | | EP 21 30 5029 |
| | Office européen des brevets | | |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 910 237 A1 (SAKAI CHEMICAL INDUSTRY CO [JP]) 26 August 2015 (2015-08-26) * the whole document * | 1-15 | INV. A61K8/02 A61K8/19 A61Q19/08 |
| X | WO 2010/146630 A1 (SHISEIDO CO LTD [JP]; KANEMARU TETSUYA [JP]; JOICHI KYOKO [JP]) 23 December 2010 (2010-12-23) * the whole document * | 1-15 | |
| X | DATABASE WPI Week 201169 Thomson Scientific, London, GB; AN 2011-M80003 XP002803438, & JP 2011 201828 A (SHISEIDO CO LTD) 13 October 2011 (2011-10-13) * abstract * | 1-15 | |
| X | DATABASE WPI Week 199241 Thomson Scientific, London, GB; AN 1992-335306 XP002803439, & JP H04 238812 A (SAKAI CHEM IND CO LTD) 26 August 1992 (1992-08-26) * abstract * | 1-15 | TECHNICAL FIELDS SEARCHED (IPC) A61K A61Q |
| X | EP 3 517 178 A1 (OMYA INT AG [CH]) 31 July 2019 (2019-07-31) * the whole document * | 1-15 | |
| X | EP 3 517 176 A1 (OMYA INT AG [CH]) 31 July 2019 (2019-07-31) * the whole document * | 1-15 | |
| | -/-- | | |

The present search report has been drawn up for all claims

| Place of search | Date of completion of the search | Examiner |
|---|---|---|
| Munich | 24 June 2021 | Yon, Jean-Michel |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 21 30 5029

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE WPI<br>Week 201169<br>Thomson Scientific, London, GB;<br>AN 2011-M78529<br>XP002803440,<br>& JP 2011 201829 A (SHISEIDO CO LTD)<br>13 October 2011 (2011-10-13)<br>* abstract *<br>----- | 1-15 | |
| X | WO 2017/127362 A1 (SUN CHEMICAL CORP [US])<br>27 July 2017 (2017-07-27)<br>* the whole document *<br>----- | 1-15 | |

TECHNICAL FIELDS
SEARCHED      (IPC)

The present search report has been drawn up for all claims

| Place of search | Date of completion of the search | Examiner |
|---|---|---|
| Munich | 24 June 2021 | Yon, Jean-Michel |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 30 5029

This annex lists the patent family members relating to the patent documents cited in the above-mentioned European search report.
The members are as contained in the European Patent Office EDP file on
The European Patent Office is in no way liable for these particulars which are merely given for the purpose of information.

24-06-2021

| Patent document cited in search report | | Publication date | Patent family member(s) | | Publication date |
|---|---|---|---|---|---|
| EP 2910237 | A1 | 26-08-2015 | CN | 104822359 A | 05-08-2015 |
| | | | EP | 2910237 A1 | 26-08-2015 |
| | | | JP | 6269498 B2 | 31-01-2018 |
| | | | JP | WO2014061689 A1 | 05-09-2016 |
| | | | KR | 20150067232 A | 17-06-2015 |
| | | | US | 2015290094 A1 | 15-10-2015 |
| | | | WO | 2014061689 A1 | 24-04-2014 |
| WO 2010146630 | A1 | 23-12-2010 | NONE | | |
| JP 2011201828 | A | 13-10-2011 | NONE | | |
| JP H04238812 | A | 26-08-1992 | JP | 3013445 B2 | 28-02-2000 |
| | | | JP | H04238812 A | 26-08-1992 |
| EP 3517178 | A1 | 31-07-2019 | AR | 114314 A1 | 19-08-2020 |
| | | | AU | 2019211008 A1 | 11-06-2020 |
| | | | BR | 112020010919 A2 | 17-11-2020 |
| | | | CN | 111629790 A | 04-09-2020 |
| | | | EP | 3517178 A1 | 31-07-2019 |
| | | | EP | 3743164 A1 | 02-12-2020 |
| | | | JP | 2021510686 A | 30-04-2021 |
| | | | KR | 20200116119 A | 08-10-2020 |
| | | | US | 2020397672 A1 | 24-12-2020 |
| | | | WO | 2019145372 A1 | 01-08-2019 |
| EP 3517176 | A1 | 31-07-2019 | AR | 114315 A1 | 19-08-2020 |
| | | | AU | 2019211005 A1 | 11-06-2020 |
| | | | BR | 112020010909 A2 | 17-11-2020 |
| | | | CN | 111655342 A | 11-09-2020 |
| | | | EP | 3517176 A1 | 31-07-2019 |
| | | | EP | 3743160 A1 | 02-12-2020 |
| | | | JP | 2021510690 A | 30-04-2021 |
| | | | KR | 20200116128 A | 08-10-2020 |
| | | | TW | 201932094 A | 16-08-2019 |
| | | | US | 2021077368 A1 | 18-03-2021 |
| | | | WO | 2019145369 A1 | 01-08-2019 |
| JP 2011201829 | A | 13-10-2011 | NONE | | |
| WO 2017127362 | A1 | 27-07-2017 | CN | 108601711 A | 28-09-2018 |
| | | | EP | 3405165 A1 | 28-11-2018 |
| | | | JP | 2019502733 A | 31-01-2019 |
| | | | US | 2018344586 A1 | 06-12-2018 |
| | | | WO | 2017127362 A1 | 27-07-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03004414 A
- US 6610261 B, Michelin